# EUROPÄISCHE PATENTSCHRIFT

(11) **EP 1 626 684 B1**
(45) Veröffentlichungstag und Bekanntmachung des Hinweises auf die Patenterteilung: **19.12.2007**
(21) Anmeldenummer: 03709550.2
(22) Anmeldetag: 14.04.2003
(51) Int. Cl.: A61F 2/44

(54) **ZWISCHENWIRBELIMPLANTAT**
INTERVERTEBRAL IMPLANT
IMPLANT INTERVERTEBRAL

(43) Veröffentlichungstag der Anmeldung: 22.02.2006
(73) Patentinhaber: Synthes GmbH, 4436 Oberdorf (CH)
(72) Erfinder: BAUMGARTNER, Daniel, CH-4702 Oensingen (CH); WYMANN, Martin, CH-3097 Liebefeld (CH); GAGO HO, Mario, CH-4500 Solothurn (CH); BURRI, Adrian, CH-3900 Brig (CH)
(74) Vertreter: Lusuardi, Werther
(86) Internationale Anmeldenummer: PCT/CH2003/000247
(87) Internationale Veröffentlichungsnummer: WO 2004/089257

(56) Entgegenhaltungen:
- EP-A- 0 346 129
- WO-A-02/17825
- WO-A-93/16664
- US-A- 3 867 728
- US-A1- 2003 045 939
- US-B1- 6 419 704

## Beschreibung

Die Erfindung bezieht sich auf Zwischenwirbelimplantat, gemäss dem Oberbegriff des Patentanspruchs 1.

Eine gattungsgemässe Bandscheibenprothese ist aus der US 4,911,718 LEE bekannt. Diese bekannte Bandscheibenprothese besteht aus einem zentralen Kern, welcher aus einem biokompatiblen Elastomer so geformt ist, dass er annähernd dem Nucleus pulposus einer natürlichen Bandscheibe entspricht, sowie aus einem um den Kern angeordneten mehrschichtigen Laminat aus in einem Elastomer gebundenen Fibern. Jede der Laminatschichten verfügt über ein eigenes Fadensystem, so dass eine Mehrzahl von Fiber-Gruppen vorliegt. Die einzelnen Schichten weisen eine verschiedene Orientierung der Fibern auf, wobei die Winkel der Fibern relativ zur Zentralachse der Bandscheibenprothese in einem Bereich zwischen ±20° und ±50° vorzugsweise 0°, +45° und -45° betragen.

Aus der WO90/00374 KLAUE ist im weiteren eine Hüftendoprothese bekannt, deren Schaft aus einem rohrförmigen Geflecht besteht, d.h. einem Gebilde, welches aus mindestens zwei gegeneinander gekreuzten Serien von Fasern besteht. Das Innere des rohrförmigen Geflechtes bleibt bei dieser Anwendung als Femurschaft-Komponente leer.

Bei der in der US 4,911,718 LEE offenbarten Prothese sind die einzelnen Fasern zwar im Laminat, welches aus einem Elastomer oder einer anderen Art von Kunststoff besteht, integriert, sind aber an ihren Enden nur an den Endplatten angeklebt, so dass sie den Kern nicht umschliessen und deshalb, bei einer radialen Ausdehnung des Kerns keine Zugkräfte aufnehmen können. Beim Ankleben der aus dem Matrix-Faserverbund ausgeschnittenen Seitenwände an die Endplatte ist eine Fixation der integrierten Faser selber an der Endplatte recht schwierig, bietet doch nur der Querschnitt der Faser eine Kontaktfläche für den chemischen Verbund. Speziell an diesen Verbindungsstellen der Fasern an die Endplatten treten deshalb erhöhte Spannungswerte auf.

Im weiteren haben die einzelnen Fasern bei LEE nur eine Länge von der unteren zur oberen Deckplatte, was der Mantelhöhe oder einer Diagonalen der projizierten Mantelfläche entspricht. Die auftretenden Kräfte können somit nur entlang dieser Längen infolge Scherkraftübertragung der Faser an das Elastomer abgebaut werden. Orte erhöhter Spannungen resultieren so an den Einspannungen, also an den Faserenden.

Die in der WO 90/00374 KLAUE offenbarte Prothese umfasst ein Fasersystem, dessen einzelne Fasern nicht an beiden Enden fixiert sind, sowie keinen deformierbaren Kern. Bei einer axialen Kompression der Prothese lassen sich daher die auftretenden axialen Druckkräfte nicht in Zugkräfte auf die Fasern übertragen.

Aus der US-A 3,867,728 STUBSTAD ET AL. ist eine gattungsgemässe Bandscheibenprothese bekannt, welche eine elastomere Sandwich-Struktur mit einem Fasersystem aufweist. Nachteilig bei dieser bekannten Prothese ist, dass das mit den Deckplatten verbundene Fasersystem entweder nicht in einen Mantelkörper eingebettet ist oder in einer anderen Ausführungsform in einem mehrschichtigen Laminat aus einem Elastomer eingebettet ist.

Hier will die Erfindung Abhilfe schaffen. Der Erfindung liegt die Aufgabe zugrunde, ein Zwischenwirbelimplantat zu schaffen, welches ein mit den Deckplatten verbundenes Fasersystem umfasst, wodurch ein das Mittelteil umschliessender, aus einem homogenen Material bestehender Mantelkörper verstärkt wird.

Die Erfindung löst die gestellte Aufgabe mit einem Zwischenwirbelimplantat, welches die Merkmale des Anspruchs 1 aufweist.

Die durch die Erfindung erreichten Vorteile sind im wesentlichen darin zu sehen, dass dank des erfindungsgemässen Zwischenwirbelimplantates
- das Fasersystem zuerst um das Mittelteil gewickelt werden kann und anschliessend in ein den elastischen Mantelkörper bildendes Elastomer eingegossen werden kann, so dass der das Mittelteil umschliessende Mantel einfach herstellbar ist;
- durch das Anbringen des elastischen Materials um das Fasersystem nach dessen Wicklung die Verankerung des Fasersystemes auf verschiedene Arten, beispielsweise auch an den gegeneinander gerichteten, inneren Oberflächen der Deckplatten möglich ist;
- das Mittelteil eine Bewegung der beiden angrenzenden Wirbelkörper im Falle einer Kompression, einer Flexion respektive Extension, einer lateralen Biegung und einer Torsion zulässt;
- das momentane Rotationszentrum oder die momentanen Drehachsen nicht vom Zwischenwirbelimplantat selbst vorgegeben werden, und sich diese nach dem Gesetz der minimal auftretenden Kräfte oder Momente einstellen können;
- durch die Variation der Anzahl Fasern in Umfangsrichtung, des Querschnitts der Fasern und der Materialwahl das Verhalten des Zwischenwirbelimplantates so einstellbar ist, dass die Bewegungen in Abhängigkeit unterschiedlich auftretender Lasten sich wie bei der natürlichen Bandscheibe verhält; und
- durch Variation der Anordnung und Ausführungsform des Fasersystemes dem Zwischenwirbelimplantat gewisse Bewegungseinschränkungen gesetzt werden können und dass ab einer gewissen Deformation ein Grenzbereich auftritt, wo trotz weiter ansteigenden Kräften keine Deformation oder bei auftretenden Momenten kein weiteres Abkippen des Implantates erfolgt.

Die bei einer Belastung der Wirbelsäule auftretenden axialen Kompressionskräfte werden über die beiden Endplatten auf das Mittelteil übertragen. Diese deformieren das zwischen den beiden Endplatten liegende Mittelteil, insbesondere einen allfällig darin vorhandenen elastischen Formkörper derart, dass sich das Mittelteil radial ausbeult. Diese Expansion des Mittelteils wird durch das das Mittelteil umschliessende Fasersystem eingeschränkt und die auftretenden, radial gerichteten Druckkräfte können vom Fasersystem als Zugkraft aufgenommen werden. Einem weiteren, nachteiligen Ausbeulen des Mittelteils kann somit Grenzen gesetzt werden. Durch die Verankerung des Fasersystems in den beiden Deckplatten bleibt das Zwischenwirbelimplantat auch bei grössten Belastungen stabil und das Fasersystem ist in der Lage, auch erheblichen Zugkräften standzuhalten.

In einer bevorzugten Ausführungsform ist das gesamte Fasersystem in den elastischen Mantelkörper eingebettet, so dass das Fasersystem nicht notwendigerweise aus einem biokompatiblen Material bestehen muss.

In einer weiteren Ausführungsform ist das Fasersystem nur teilweise in den elastischen Mantelkörper eingebettet, wobei das Fasersystem eine bezüglich der Zentralachse radiale Dicke δ und der elastische Mantelkörper eine radiale Dicke d aufweist, und das Verhältnis von δ/d x 100% in einem Bereich zwischen 80 % und 350 % liegt. Dadurch ist der Vorteile erreichbar, dass die bei einer Flexions-/Extensionsbewegung oder lateralen Beugung der angrenzenden Wirbelkörper auftretenden grossen Relativbewegungen im peripheren Bereich der Deckplatten keinem grossen Widerstand durch den elastischen Mantelkörper ausgesetzt sind und dadurch die Gefahr einer Rissbildung im Mantelkörper geringer ist.

Die Einbettung des Fasersystems in den elastischen Mantelkörper kann in verschiedenen Ausführungsformen derart ausgebildet sein, dass
a) das Fasersystem relativ zum elastischen Material des Mantelkörpers bewegbar ist; oder
b) das Fasersystem relativ zum elastischen Material des Mantelkörpers unbewegbar ist.

In wiederum einer weiteren Ausführungsform ist das gesamte Fasersystem an den Deckplatten verankert, so dass grössere Zugkräfte durch das Fasersystem übernommen werden können und das Zwischenwirbelimplantat dadurch eine hohe Torsionssteifigkeit erhält.

In einer anderen Ausführungsform besteht der das Fasersystem aufnehmende Mantelkörper aus einem elastischen, biokompatiblen Material, vorzugsweise einem Elastomer, insbesondere auf Basis von Polyurethan (PUR) hergestellt. Es können aber auch Silikonkautschuk, Polyethylen, Polycarbonaturethan (PCU) oder Polyethylenterephthalat (PET) verwendet werden.

In wiederum einer weiteren Ausführungsform ist das Mittelteil mindestens teilweise mit einem inkompressiblen Medium, vorzugsweise einer Flüssigkeit gefüllt.

In einer anderen Ausführungsform umfasst das Mittelteil einen inkompressiblen Flüssigkeitskern und einen darum herum angeordneten, elastischen Formkörper, wobei die Flüssigkeit beispielsweise in einer im Formkörper angeordneten Kavität aufgenommen werden kann. Damit ist der Vorteil erzielbar, dass durch den Flüssigkeitskern ein mechanisches Verhalten des Zwischenwirbelimplantates analog zu einer physiologischen Bandscheibe erreicht wird. Durch die axiale Deformation des elastischen Mittelteiles kommt es zu einer radialen Ausdehnung der inkompressiblen Flüssigkeit und in der Folge zu einer radialen Ausdehnung der das Fasersystem enthaltenden Wand des Mittelteiles. Die durch die radiale Ausdehnung, respektive Ausbeulung der Wand des Mittelteiles auftretenden Zugkräfte werden im wesentlichen durch die Fasern aufgenommen.

Die Verankerung der Fasern an den Deckplatten kann beispielsweise auf folgende Arten erfolgen:
a) mechanisch durch Führung der als Endlosfasern ausgestalteten Fasern durch Nuten und über die aussenstehenden Oberflächen der Deckplatten von einer Nute zu einer anderen Nute. Die Fasern umschliessen so das Mittelteil zusammen mit den Deckplatten. Durch die Führung der Fasern in den Nuten wird das Fasersystem so an den Deckplatten verankert, dass bei auf die Fasern wirkenden Zugkräfte kein Verrutschen der Fasern an den Seitenflächen möglich ist, da die Fasern nur Zugkräfte aufnehmen können;
b) mechanisch durch keilförmige Ausgestaltung der Nuten, so dass die sich von Deckplatte zu Deckplatte erstreckenden Fasern in den Nuten festklemmbar sind; und/oder
c) durch Verkleben des Fasersystemes an den Deckplatten.

In wiederum einer anderen Ausführungsform des erfindungsgemässen Zwischenwirbelimplantates umfasst jede Deckplatte an ihrer Peripherie eine Seitenfläche und auf dem Umfang verteilt, radial in die Seitenflächen eindringende Nuten. Die zum Fasersystem gehörenden Fasern werden durch diese Nuten geführt.

In einer weiteren Ausführungsform ist das Mittelteil und das Fasersystem formschlüssig mit den Deckplatten verbunden.

Bei Verwendung einer Endlosfaser, die das gesamte Implantat umspannt, verteilen sich die Spannungen vorteihafterweise auf den Gesamtumfang dieser Umwicklung. Vorzugsweise ist das Fasersystem in Form eines Gewirkes, Gewebes oder Gestrickes gebildet.

In einer anderen Ausführungsform sind Kanäle zur Aufnahme des Fasersystemes in die aussenstehenden Oberflächen der Deckplatten eingelassen.

In wiederum einer anderen Ausführungsform ist das Mittelteil im wesentlichen hohlzylindrisch, hohlprismatisch oder ein Form eines Rotationskörpers, eines Ellipsoides, einer Teilkugel oder einer Tonnenform mit zur Zentralachse koaxialer Rotationsachse ausgebildet. Durch solche Ausgestaltungen ist der Vorteil erreichbar, dass das die Lage der Rotationsachsen der angrenzenden Wirbelkörper möglichst weitgehend derjenigen der natürlichen Bandscheibe entspricht.

Das Fasersystem kann beispielsweise aus UHMWPE (Ultra High Molecular Weight Polyethylene) oder auch aus PET (Polyethylenterephthalat) bestehen.

In einer weiteren Ausführungsform des erfindungsgemässen Zwischenwirbelimplantates ist an jeder Deckplatte eine zur Anlage an die Grundplatte bzw. Deckplatte der angrenzenden Wirbelkörper bestimmte Abschlussplatte befestigt, wovon jede eine aussenstehende, quer zur Zentralachse angeordnete Oberfläche mit einer makroskopischen Strukturierung aufweist. Die Strukturierung kann beispielsweise in Form von Zähnen realisiert sein. Die makroskopische Strukturierung gestattet eine primäre Stabilisierung des Zwischenwirbelimplantates direkt nach der Operation. Damit ist eine mechanische Verankerung des Zwischenwirbelimplantates zu einem Zeitpunkt, wo das Anwachsen der Knochen an das Zwischenwirbelimplantat noch nicht stattgefunden hat, erreichbar.

In wiederum einer weiteren Ausführungsform wird das Gewebe aus ersten und zweiten Fasern gebildet, wobei die ersten Fasern einen Winkel α mit der Zentralachse einschliessen und die zweiten Fasern einen Winkel β mit der Zentralachse einschliessen. Vorzugsweise betragen die Winkel α respektive β zwischen 15° und 60°.

In einer anderen Ausführungsform sind die ersten und zweiten Fasern miteinander verflochten.

In wiederum einer anderen Ausführungsform weist der elastische Formkörper eine zur Zentralachse orthogonale Querschnittsfläche F_{F} auf, während das Mittelteil eine zur Zentralachse orthogonale Querschnittsfläche F_{M} aufweist und das Verhältnis F_{F}/F_{M} dieser beiden Querschnittsflächen zwischen 30% und 65% beträgt.

In einer weiteren Ausführungsform ist der elastische Formkörper von einer semipermeablen Membran umschlossen, wobei im Inneren des elastischen Formkörpers vorzugsweise physiologische Kochsalzlösung vorhanden ist.

Das Fasersystem kann bezüglich der Zentralachse einlagig oder mehrlagig, vorzugsweise 2- bis 6-lagig angeordnet sein. Ferner kann das Fasersystem auf dem elastischen Formkörper aufgewickelt sein. Die Aufwicklung auf dem elastischen Formkörper kann in zwei verschiedenen Richtungen, vorzugsweise in rotationssymmetrischer Anordnung erfolgen.

In wiederum einer weiteren Ausführungsform ist an jeder Deckplatte eine Abschlussplatte befestigbar, welche eine aussenstehende, quer zur Zentralachse angeordnete Oberfläche mit einer makroskopischen Strukturierung aufweist, vorzugsweise in Form von Zähnen.

Die Fasern weisen einen Durchmesser in einem Bereich zwischen 0,005 mm und 0,025 mm auf. Vorzugsweise wird aus mehreren Fasern ein Faden hergestellt (Roving), wobei beispielsweise 500 - 2000 Fasern einen Faden mit einer Querschnittsfläche von 0,5 mm² bis 2 mm² bilden.

Bei denjenigen Ausführungsformen, bei welchen das Fasersystem zueinander gekreuzte Faserabschnitte aufweist, werden bei Flexionsbewegungen des Patienten (Flexion, Extension, Laterale Flexion) die einen Faserabschnitte einseitig gespannt und bei Scherung nehmen die tangential zur Scherrichtung verlaufenden Faserabschnitte die Kräfte auf.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Seitenansicht einer Ausführungsform des erfindungsgemässen Zwischenwirbelimplantates;
Fig. 2 eine Aufsicht auf die in Fig. 1 dargestellte Ausführungsform des erfindungsgemässen Zwischenwirbelimplantates;
Fig. 3 eine Seitenansicht einer anderen Ausführungsform des erfindungsgemässen Zwischenwirbelimplantates;
Fig. 4 einen Schnitt durch die in Fig. 3 dargestellte Ausführungsform des erfindungsgemässen Zwischenwirbelimplantates;
Fig. 5a eine perspektivische Darstellung des Fasersystems einer Ausführungsform des erfindungsgemässen Zwischenwirbelimplantates;
Fig. 5b eine Draufsicht auf das in Fig. 5a dargestellte Fasersystem;
Fig. 6a eine perspektivische Darstellung des Fasersystems einer Ausführungsform des erfindungsgemässen Zwischenwirbelimplantates;
Fig. 6b eine Draufsicht auf das in Fig. 6a dargestellte Fasersystem; und
Fig. 7 einen Schnitt durch eine weitere Ausführungsform des erfindungsgemässen Zwischenwirbelimplantates.

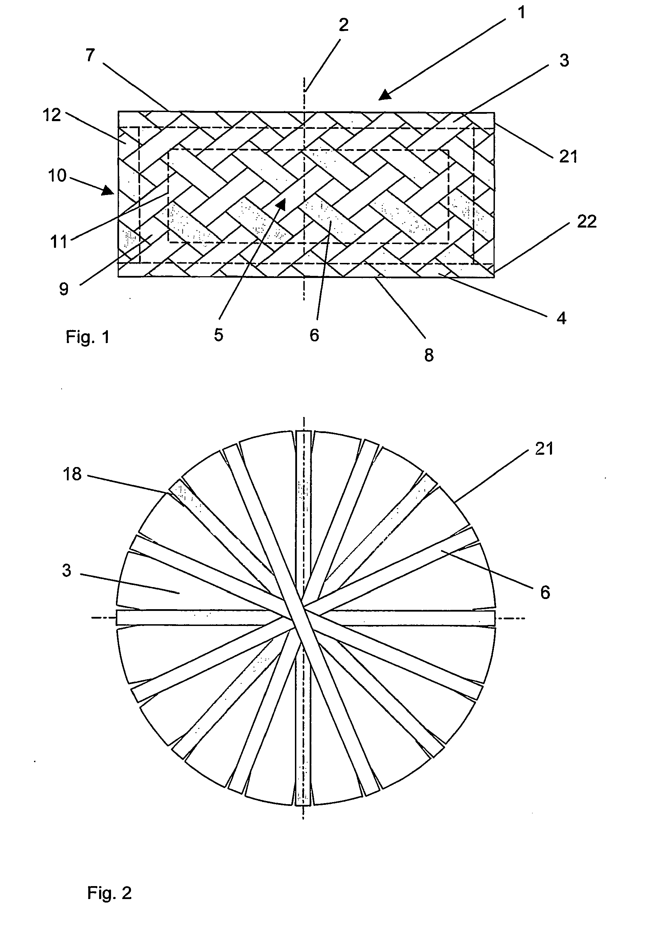

In den Fig. 1 und 2 ist eine Ausführungsform des erfindungsgemässen Zwischenwirbelimplantates 1 dargestellt, welche eine obere Deckplatte 3 und eine untere Deckplatte 4 mit je einer aussenstehenden, quer zur Zentralachse 2 verlaufende Oberfläche 7;8 und peripher je einer Seitenfläche 21;22 umfasst. Zwischen den Deckplatten 3;4 ist ein Mittelteil 10 mit einer zentralen Kavität 11 und einem Mantel 12 angeordnet, welcher das Fasersystem 5 umfasst. Zur Verankerung der zum Fasersystem 5 gehörenden Fasern 6 an den Deckplatten 3;4 umfasst jede der peripheren Seitenflächen 21;22 auf dem Umfang verteilt, radial in die Seitenflächen 21;22 eindringende Nuten 18, so dass das Fasersystem 5 in diesen Nuten 18 verankerbar ist. In der zentralen Kavität 11 ist ein elastisch deformierbarer Formkörper 9 mit einem inkompressiblen Kern, vorzugsweise ein Flüssigkeitskern 13 angeordnet. Durch die Inkompressibilität des Flüssigkeitskerns 13 wird beispielsweise bei einer zur Längsachse 2 parallelen Kompression der Deckplatten 3;4 der elastische Formkörper 9 und der Mantel 12 mit dem Fasersystem 5 radial, d.h. quer zur Längsachse 2 ausgebeult, wodurch die Fasern 6 auf Zug beansprucht werden.

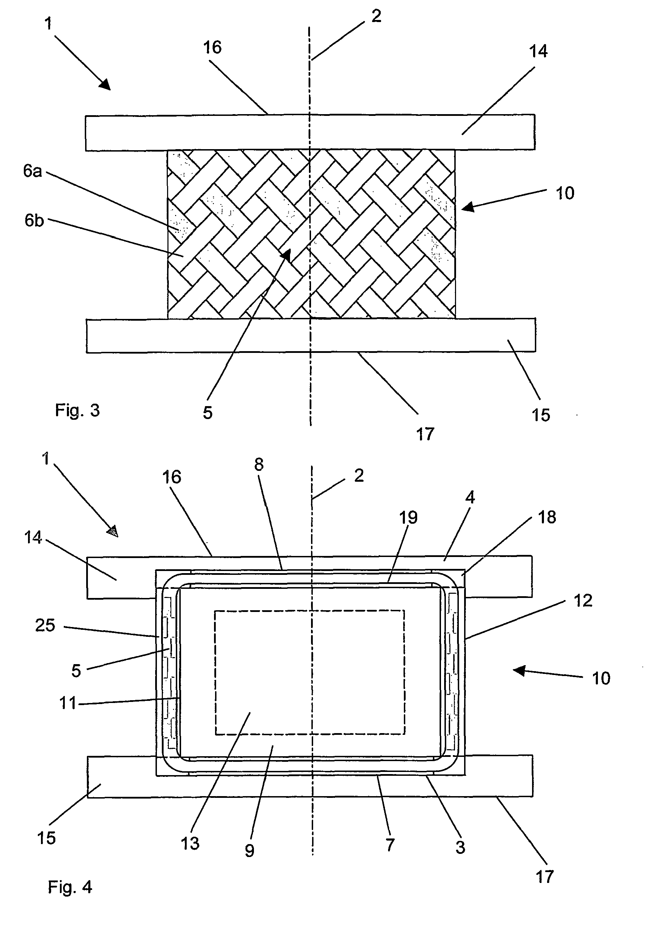

In den Fig. 3 und 4 ist eine Ausführungsform des erfindungsgemässen Zwischenwirbelimplantates 1 dargestellt, welche zwei quer zur Zentralachse 2 angeordnete Deckplatten 3;4 und ein dazwischen liegendes, elastisch deformierbares Mittelteil 10 umfasst. Das Mittelteil 10 umfasst einen zur Zentralachse 2 koaxialen hohlzylindrischen Mantel 12 und eine zentrale Kavität 11. In der zentralen Kavität 11 angeordnet ist ein elastischer Formkörper 9 mit einem inkompressiblen Kern, vorzugsweise einem Flüssigkeitskern 13. Der Formkörper 9 ist mit einer semipermeablen Membran umschlossen, während der Mantel 12, welcher das Fasersystem 5 und einen elastischen, vom Fasersystem 5 durchzogenen Mantelkörper 25 umfasst, aus einem Kunststoff gefertigt ist. Die Abschlussplatten 14; 15 sind fest mit den Deckplatten 3;4 verbunden und weisen axial aussenstehende Oberflächen 16;17 auf, welche an den Endplatten zweier benachbarter Wirbelkörper zu Anlage bringbar sind. Das Fasersystem 5 ist an den Deckplatten 3;4 verankert und in den Mantel 12 integriert und dient dazu, die Kräfte auf das Mittelteil 10 aufzunehmen, welche durch die an die Abschlussplatten 14;15 angrenzenden Wirbelkörper auf das Zwischenwirbelimplantat 1 ausgeübt werden, d.h. Torsionskräfte durch Verdrehen der Wirbelkörper um die Zentralachse 2 relativ zueinander oder Biegemomente durch laterale Beugung und/oder Flexion/Extension der Wirbelsäule. Beispielsweise wird eine zur Zentralachse 2 parallele Kompressionskraft auf das Zwischenwirbelimplantat 1 von den beiden Abschlussplatten 14;15 über die beiden Deckplatten 3;4 auf das Mittelteil 10 übertragen, wobei in der Folge der elastische Formkörper 9 quer zur Zentralachse 2 ausgebeult wird. Diese Expansionsbewegung des elastischen Formkörpers 9 wird auf den Mantel 12 mit dem Fasersystem 5 übertragen und durch dieses eingeschränkt. Da das Fasersystem 5 an den Deckplatten 3;4 verankert ist, bewirkt die quer zur Zentralachse 2 wirkende Druckkraft Zugkräfte auf die Fasern des Fasersystems 5. Das Fasersystem 5 besteht hier aus Kunststoffasern, vorzugsweise aus UHMWPE-Fasern (Ultra High Molecular Weight Polyethylene) oder aus PET (Polyethylterephthalat) und umfasst ein Geflecht aus ersten und zweiten Fasern 6a;6b, welche miteinander verflochten sind. Dabei schliessen die ersten Fasern 6a einen Winkel α und die zweiten Fasern 6b einen Winkel β mit der Zentralachse 2 ein. In der hier dargestellten Ausführungsform des erfindungsgemässen Zwischenwirbelimplantates 1 sind die Winkel α und β gleich gross und betragen zwischen 15° und 60°. An den Deckplatten 3;4 verankert sind die Fasern 6a;6b mittels Nuten 18, welche parallel zur Zentralachse 2 am Umfang der Deckplatten 3;4 angeordnet sind, so dass die Fasern 6a;6b durch die Nuten 18 durchgeführt und können über den Oberflächen 7;8 in einem Kanal 19 zur nächsten Nute 18 geführt werden. Die Deckplatten 3;4 sind aus einem Kunststoff gefertigt, während die aussen angeordneten Abschlussplatten 14;15 aus Titan oder einer Titanlegierung hergestellt sind. Die aussen angeordneten Abschlussplatten 14;15 sind entweder form- oder reibschlüssig mit den Deckplatten 3;4 verbunden. Insbesondere können sie untereinander verklebt oder verschweisst sein.

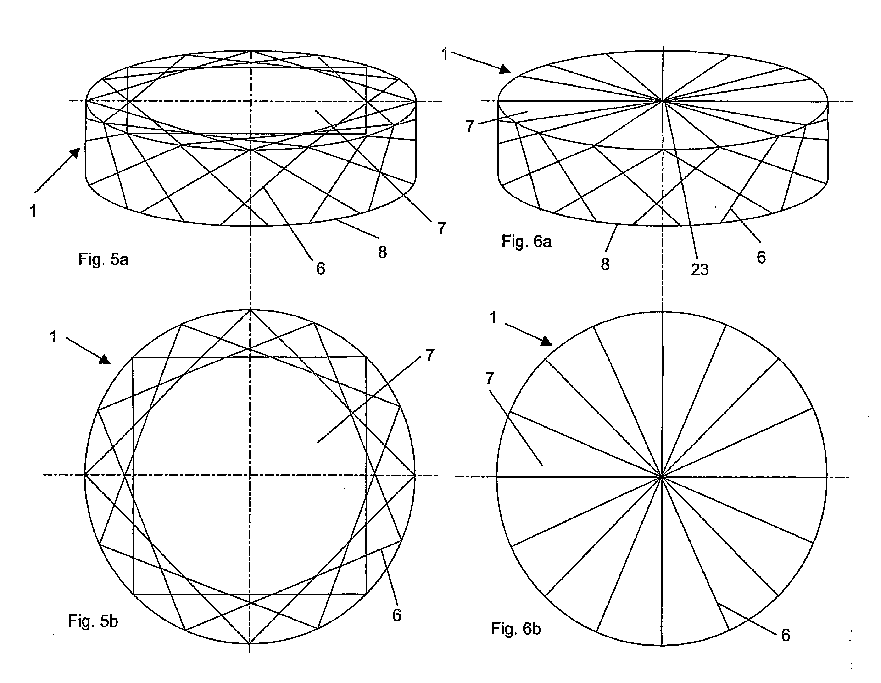

In den Fig. 5a und 5b ist ein Fasersystem 5 gemäss einer Ausführungsform des erfindungsgemässen Zwischenwirbelimplantates 1 dargestellt, wo die über die Endplatten 3;4 verlaufenden Fasern 6 Sehnen auf den kreisförmigen Oberflächen 7;8 der Deckplatten 3;4 bilden.

In den Fig. 6a und 6b ist ein Fasersystem 5 gemäss einer Ausführungsform des erfindungsgemässen Zwischenwirbelimplantates 1 dargestellt, wo die über die Endplatten 3;4 verlaufenden Fasern 6 sich beim Durchstosspunkt von Zentralachse 2 und Endplatte 3;4 kreuzen.

Gegenüber der diagonalen Anordnung der Fasern 6 (Fig. 6a;6b) hat die Führung der Fasern 6 als Sehnen (Fig. 5a;5b) über den Oberflächen 7;8 der Endplatten 3;4 die folgenden Vorteile:
- aufgrund der besseren Verteilung der Kreuzungspunkte der Fasern 6 kommt es zu keiner Anhäufung, insbesondere zwischen den aussenstehenden Oberflächen 7;8 der Deckplatten 3;4 und den Abschlussplatten 14;15 (Fig. 3 und 4); und
- das Fasersystem 5 lässt sich mit Hilfe einer Wickeltechnik zur Zentralachse 2 symmetrisch herstellen, wobei das Zwischenwirbelimplantat 1 in den Schnittpunkten zwischen der Zentralachse 2 und den Deckplatten (3;4) eingespannt werden kann.

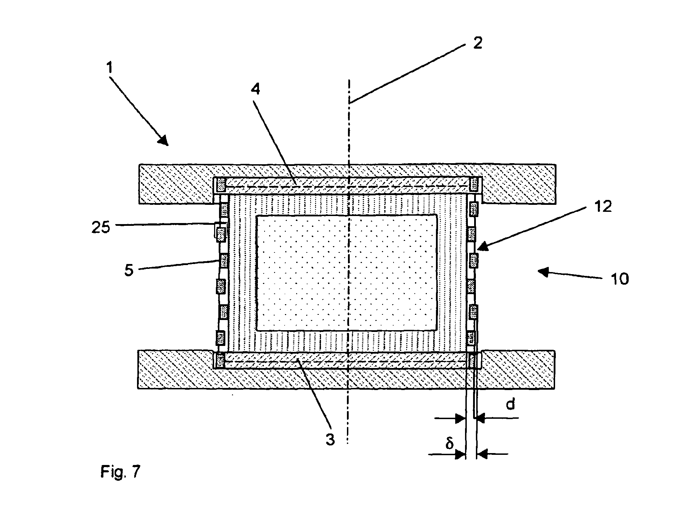

In Fig. 7 dargestellt ist eine Ausführungsform, welche sich von der in den Fig. 3 und 4 dargestellten Ausführungsform nur darin unterscheidet, dass der peripher am Mittelteil 10 angeordnete Mantel 12 einen elastischen, vom Fasersystem 5 nur teilweise durchzogenen Mantelkörper 25 umfasst, dessen Dicke d kleiner als die radiale Dicke δ des Fasersystems 5 ist.

## Patentansprüche

1. Zwischenwirbelimplantat (1) mit einer Zentralachse (2) umfassend
A) eine untere Deckplatte (3) und eine obere Deckplatte (4), welche je eine aussenstehende, quer zur Zentralachse (2) verlaufende Oberfläche (7;8) aufweisen;
B) ein zwischen den Deckplatten (3;4) angeordnetes Mittelteil (10) mit einem Mantel (12), welcher ein Fasersystem (5) umfasst, wobei
C) das Fasersystem (5) mindestens teilweise mit den Deckplatten (3;4) verbunden ist,
**dadurch gekennzeichnet, dass**
D) das Fasersystem (5) über die aussenstehenden Oberflächen (7;8) der beiden Deckplatten (3;4) geführt wird und das Mittelteil sowie die beiden Deckplatten (3;4) mindestens teilweise umschliesst; und
E) der Mantel (12) einen das Mittelteil (10) peripher umschliessenden, elastischen Mantelkörper (25) umfasst, welcher aus einem homogenen Material besteht und vom Fasersystem (5) durchzogen wird.

2. Zwischenwirbelimplantat (1) nach Anspruch 1, **dadurch gekennzeichnet, dass** das gesamte Fasersystem (5) in den elastischen Mantelkörper (25) eingebettet ist.

3. Zwischenwirbelimplantat (1) nach Anspruch 1, **dadurch gekennzeichnet, dass** das Fasersystem (5) nur teilweise im Mantelkörper (25) eingebettet ist.

4. Zwischenwirbelimplantat (1) nach Anspruch 3, **dadurch gekennzeichnet, dass** das Fasersystem (5) bezüglich der Zentralachse (2) eine radiale Dicke δ und der Mantelkörper (25) eine radiale Dicke d aufweist, wobei das Verhältnis von δ/d x 100% in einem Bereich zwischen 80 % und 350 % liegt.

5. Zwischenwirbelimplantat (1) nach einem der Ansprüche 1 bis 4, **dadurch gekennzeichnet, dass** das Fasersystem (5) relativ zum Mantelkörper (25) bewegbar ist.

6. Zwischenwirbelimplantat (1) nach einem der Ansprüche 1 bis 4, **dadurch gekennzeichnet, dass** das Fasersystem (5) relativ zum Mantelkörper (25) unbewegbar gelagert ist.

7. Zwischenwirbelimplantat (1) nach einem der Ansprüche 1 bis 6, **dadurch gekennzeichnet**, das gesamte Fasersystem (5) mit den Deckplatten (3;4) verbunden ist.

8. Zwischenwirbelimplantat (1) nach einem der Ansprüche 1 bis 7, **dadurch gekennzeichnet, dass** der Mantelkörper (25) aus einem elastischen, biokompatiblen Material, vorzugsweise aus einem Elastomer, insbesondere auf Basis von Polyurethan, oder Silikonkautschuk, Polyethylen, Polycarbonaturethan oder Polyethylenterephtalat besteht.

9. Zwischenwirbelimplantat (1) nach einem der Ansprüche 1 bis 8, **dadurch gekennzeichnet, dass** das Mittelteil (10) mindestens teilweise mit einem inkompressiblen Medium gefüllt ist.

10. Zwischenwirbelimplantat (1) nach Anspruch 9, **dadurch gekennzeichnet, dass** das inkompressible Medium eine Flüssigkeit ist.

11. Zwischenwirbelimplantat (1) nach Anspruch 10, **dadurch gekennzeichnet, dass** das Mittelteil (10) einen inkompressiblen Flüssigkeitskern (13) und einen darum herum angeordneten elastischen Formkörper (9) umfasst.

12. Zwischenviürbelimplantat (1) nach einem der Ansprüche 1 bis 11, **dadurch gekennzeichnet, dass** das Mittelteil (10) eine Kavität (11) aufweist.

13. Zwischenwirbelimplantat (1) nach einem der Ansprüche 1 bis 12, **dadurch gekennzeichnet, dass** das Fasersystem (5) an oder in den Deckplatten (3;4) mechanisch verankert ist.

14. Zwischenwirbelimplantat (1) nach einem der Ansprüche 1 bis 12, **dadurch gekennzeichnet, dass** das Fasersystem (5) mit den Deckplatten (3;4) verklebt ist.

15. Zwischenwirbelimplantat (1) nach einem der Ansprüche 1 bis 12, **dadurch gekennzeichnet, dass** das Mittelteil (10) mit dem integrierten Fasersystem (5) formschlüssig mit den Deckplatten (3;4) verbunden ist.

16. Zwischenwirbelimplantat (1) nach einem der Ansprüche 1 bis 15, **dadurch gekennzeichnet, dass** das Fasersystem (5) durch eine Endlosfaser, vorzugsweise in Form eines Gewirkes oder Gestrickes gebildet wird.

17. Zwischenwirbelimplantat (1) nach einem der Ansprüche 1 bis 16, **dadurch gekennzeichnet, dass** jede Deckplatte (3;4) an ihrer Peripherie eine Seitenfläche (21;22) und auf dem Umfang verteilt, radial in die Seitenflächen (21;22) eindringende Nuten (18) umfasst, und das Fasersystems (5) in diesen Nuten (18) verankerbar ist.

18. Zwischenwirbelimplantat (1) nach einem der Ansprüche 1 bis 17, **dadurch gekennzeichnet, dass** in die aussenstehenden Oberflächen (7;8) der Deckplatten (3;4) Kanäle (19) zur Aufnahme des Fasersystems (5) eingelassen sind.

19. Zwischenwirbelimplantat (1) nach einem der Ansprüche 1 bis 18, **dadurch gekennzeichnet, dass** das Fasersystem (5) durch ein Gewebe gebildet wird.

20. Zwischenwirbelimplantat (1) nach einem der Ansprüche 1 bis 19, **dadurch gekennzeichnet, dass** das Mittelteil (10) im wesentlichen hohlzylindrisch, hohlprismatisch oder in Form eines Rotationskörpers, eines Ellipsoides, einer Teilkugel oder einer Tonnenform mit zur Zentralachse (2) koaxialer Rotationsachse ausgebildet ist:

21. Zwischenwirbelimplantat (1) nach Anspruch 19 oder 20 **dadurch gekennzeichnet, dass** das Gewebe aus ersten und zweiten Fasern (6a/6b) gebildet wird und die ersten Fasern (6a) einen Winkel α mit der Zentralachse (2) einschliessen und die zweiten Fasern (6b) einen Winkel β mit der Zentralachse (2) einschliessen.

22. Zwischenwirbelimplantat (1) nach Anspruch 21, **dadurch gekennzeichnet, dass** die ersten und zweiten Fasern (6a;6b) miteinander verflochten sind.

23. Zwischenwirbelimplantat (1) nach einem der Ansprüche 11 bis 22, **dadurch gekennzeichnet, dass** der elastische Formkörper (9) eine zur Zentralachse (2) orthogonale Querschnittsfläche F_{F} aufweist, das Mittelteil (10) eine zur Zentralachse (2) orthogonale Querschnittsfläche F_{M} aufweist und das Verhältnis F_{F}/F_{M} dieser beiden Querschnittsflächen zwischen 30% und 65% beträgt.

24. Zwischenwirbelimplantat (1) nach einem der Ansprüche 21 bis 23, **dadurch gekennzeichnet, dass** der Winkel α zwischen 15° und 60° beträgt.

25. Zwischenwirbelimplantat (1) nach einem der Ansprüche 21 bis 24, **dadurch gekennzeichnet, dass** der Winkel β zwischen 15° und 60° beträgt.

26. Zwischenwirbelimplantat (1) nach einem der Ansprüche 11 bis 25, **dadurch gekennzeichnet, dass** der elastische Formkörper (9) von einer semipermeablen Membran umschlossen ist und im Inneren des elastischen Formkörpers (9) vorzugsweise physiologische Kochsalzlösung vorhanden ist.

27. Zwischenwirbelimplantat (1) nach einem der Ansprüche 1 bis 26, **dadurch gekennzeichnet, dass** das Fasersystem (5) bezüglich der Zentralachse (2) einlagig angeordnet ist.

28. Zwischenwirbelimplantat (1) nach einem der Ansprüche 1 bis 26, **dadurch gekennzeichnet, dass** das Fasersystem (5) bezüglich der Zentralachse (2) mehrlagig, vorzugsweise 2- bis 6-lagig angeordnet ist.

29. Zwischenwirbelimplantat (1) nach einem der Ansprüche 11 bis 28, **dadurch gekennzeichnet, dass** das Fasersystem (5) auf dem elastischen Formkörper (9) aufgewickelt ist.

30. Zwischenwirbelimplantat (1) nach Anspruch 29, **dadurch gekennzeichnet, dass** das Fasersystem (5) in zwei verschiedenen Richtungen auf dem elastischen Formkörper (9) aufgewickelt ist, vorzugsweise in rotationssymmetrischer Anordnung.

31. Zwischenwirbelimplantat (1) nach einem der Ansprüche 1 bis 30, **dadurch gekennzeichnet, dass** das Fasersystem (5) aus UHMWPE (Ultra High Molecular Weight Polyethylene) besteht.

32. Zwischenwirbelimplantat (1) nach einem der Ansprüche 1 bis 31, **dadurch gekennzeichnet, dass** an jeder Deckplatte (3,4) eine Abschlussplatte (14;15) befestigbar ist, welche eine aussenstehende, quer zur Zentralachse (2) angeordnete Oberfläche (16;17) mit einer makroskopischen Strukturierung aufweist, vorzugsweise in Form von Zähnen.

33. Zwischenwirbelimplantat (1) nach einem der Ansprüche 1 bis 32, **dadurch gekennzeichnet, dass** die Fasern einen Durchmesser in einem Bereich zwischen 0,005 mm und 0,025 mm aufweisen.

## Claims

1. An intervertebral implant (1) with a central axis (2), comprising
A) a bottom cover plate (3) and a top cover plate (4), each with an external surface (7, 8) extending transversely to the central axis (2),
B) a central part (10) provided between the cover plates (3, 4) with a sheathing (12) which comprises a fibre system (5), wherein
C) the fibre system (5) is joined with the cover plates (3, 4) at least partially,
**characterised in that**
D) the fibre system (5) is guided over the external surfaces (7, 8) of both cover plates (3, 4) and surrounds at least partially the central part as well as both cover plates (3, 4), and
E) the sheathing (12) comprises an elastic sheathing body (25) that surrounds the central part (10) on the periphery and is made from a homogeneous material and is passed through by the fibre system (5).

2. An intervertebral implant (1) according to claim 1, **characterised in that** the entire fibre system is embedded in the elastic sheathing body (25).

3. An intervertebral implant (1) according to claim 1, **characterised in that** the fibre system is only partially embedded in the elastic sheathing body (25).

4. An intervertebral implant (1) according to claim 3, **characterised in that** the fibre system (5) has a radial thickness δ relative to the central axis (2) and the sheathing body (25) has a radial thickness d, and the δ/d x 100% ratio is in a range between 80% and 350%.

5. An intervertebral implant (1) according to any one of claims 1 to 4, **characterised in that** the fibre system (5) can move relative to the sheathing body (25).

6. An intervertebral implant (1) according to any one of claims 1 to 4, **characterised in that** the fibre system (5) is so mounted that it cannot move relative to the sheathing body (25).

7. An intervertebral implant (1) according to any one of claims 1 to 6, **characterised in that** the entire fibre system (5) is joined with the cover plates (3, 4).

8. An intervertebral implant (1) according to any one of claims 1 to 7, **characterised in that** the sheathing body is made from an elastic, biocompatible material, preferably an elastomer, in particular based on polyurethane or silicone rubber, polyethylene, polycarbonate urethane or polyethylene terephthalate.

9. An intervertebral implant (1) according to any one of claims 1 to 8, **characterised in that** the central part (10) is filled at least partially with an incompressible medium.

10. An intervertebral implant (1) according to claim 9, **characterised in that** the incompressible medium is a liquid.

11. An intervertebral implant (1) according to claim 10, **characterised in that** the central part (10) comprises an incompressible liquid core (13) and an elastic formed body (9) provided around it.

12. An intervertebral implant (1) according to any one of claims 1 to 11, **characterised in that** the central part (10) has a cavity (11).

13. An intervertebral implant (1) according to any one of claims 1 to 12, **characterised in that** the fibre system (5) is mechanically anchored on or in the cover plates (3, 4).

14. An intervertebral implant (1) according to any one of claims 1 to 12, **characterised in that** the fibre system (5) is adhered to the cover plates (3, 4).

15. An intervertebral implant (1) according to any one of claims 1 to 12, **characterised in that** the central part (10) with the integrated fibre system (5) is joined with the cover plates (3, 4) in a form-locking manner.

16. An intervertebral implant (1) according to any one of claims 1 to 15, **characterised in that** the fibre system (5) is formed by an endless fibre, preferably in the form of a fabric or is knitted.

17. An intervertebral implant (1) according to any one of claims 1 to 16, **characterised in that** each cover plate comprises on its periphery a lateral surface (21, 22) and grooves (18) distributed on the circumference and radially penetrating into the lateral surfaces (21, 22) and that the fibre system (5) can be anchored in these grooves (18).

18. An intervertebral implant (1) according to any one of claims 1 to 17, **characterised in that** channels (19) are mortised in the external surfaces (7, 8) of the cover plates (3, 4) to accommodate the fibre system (5).

19. An intervertebral implant (1) according to any one of claims 1 to 18, **characterised in that** the fibre system (5) is formed by a woven material.

20. An intervertebral implant (1) according to any one of claims 1 to 19, **characterised in that** the central part (10) is essentially hollow-cylindrical, hollow-prismatic or is in the form of a body of rotation, an ellipsoid, a partial sphere or barrel-shaped with an axis of rotation that is coaxial with the central axis (2).

21. An intervertebral implant (1) according to claim 19 or 20, **characterised in that** the woven material is formed from first and second fibres (6a, 6b), and the first fibres (6a) include an angle α with the central axis (2) and the second fibres (6b) include an angle β with the central axis (2).

22. An intervertebral implant (1) according to claim 21, **characterised in that** the first and second fibres (6a, 6b) are interwoven with one another.

23. An intervertebral implant (1) according to any one of claims 11 to 22, **characterised in that** the elastic formed body (9) has at right angles to the central axis (2) a cross-sectional surface F_{F}, the central part has at right angles to the central axis (2) a cross-sectional surface F_{M} and the F_{F}/F_{M} ratio of these two cross-sectional surfaces is between 30% and 65%.

24. An intervertebral implant (1) according to any one of claims 22 to 23, **characterised in that** the angle α is between 15° and 60°.

25. An intervertebral implant (1) according to any one of claims 22 to 24, **characterised in that** the angle β is between 15° and 60°.

26. An intervertebral implant (1) according to any one of claims 11 to 25, **characterised in that** the elastic formed body (9) is surrounded by a semipermeable membrane and in the interior of the elastic formed body (9) preferably physiological table salt solution is present.

27. An intervertebral implant (1) according to any one of claims 1 to 26, **characterised in that** with regard to the central axis (2) the fibre system (5) is single-layered.

28. An intervertebral implant (1) according to any one of claims 1 to 26, **characterised in that** with regard to the central axis (2) the fibre system (5) is multi-layered, preferably 2-6 layered.

29. An intervertebral implant (1) according to any one of claims 11 to 28, **characterised in that** the fibre system (5) is wound on the elastic formed body (9).

30. An intervertebral implant (1) according to claim 29, **characterised in that** the fibre system (5) is wound on the elastic formed body (9) in two different directions, preferably rotationally symmetrically.

31. An intervertebral implant (1) according to any one of claims 1 to 30, **characterised in that** the fibre system (5) is made from UHMWPE (ultra high molecular weight polyethylene).

32. An intervertebral implant (1) according to any one of claims 1 to 31, **characterised in that** a closing plate (14, 15) can be fastened on each cover plate (3, 4), the closing plate having at right angles to the central axis (2) an external surface (16, 17) with a macroscopic structure, preferably in the form of teeth.

33. An intervertebral implant (1) according to any one of claims 1 to 32, **characterised in that** the diameter of the fibres is in a range of 0,005 mm and 0,025 mm.

## Revendications

1. Implant intervertébral (1) ayant un axe central (2), comprenant
A) une plaque de recouvrement inférieure (3) et une plaque de recouvrement supérieure (4), qui présentent chacune une surface (7 ; 8) externe s'étendant transversalement à l'axe central (2) ;
B) une partie centrale (10) située entre les plaques de recouvrement (3 ; 4) qui présente une enveloppe (12) qui comprend un système de fibres (5), dans lequel
C) le système de fibres (5) est relié au moins en partie aux plaques de recouvrement (3 ; 4),
**caractérisé en ce que**
D) le système de fibres (5) est placé sur les surfaces externes (7 ; 8) des deux plaques de recouvrement (3 ; 4) et recouvre au moins en partie la partie centrale ainsi que les deux plaques de recouvrement (3 ; 4) ; et
E) l'enveloppe (12) comprend un corps d'enveloppe élastique (25) entourant la partie centrale (10) sur la périphérie, lequel se compose d'un matériau homogène et est traversé par le système de fibres (5).

2. Implant intervertébral (1) selon la revendication 1, **caractérisé en ce que** l'ensemble du système de fibres (5) est incorporé dans le corps d'enveloppe élastique (25).

3. Implant intervertébral (1) selon la revendication 1, **caractérisé en ce que** le système de fibres (5) est incorporé seulement en partie dans le corps d'enveloppe élastique (25).

4. Implant intervertébral (1) selon la revendication 3, **caractérisé en ce que** le système de fibres (5) présente une épaisseur radiale δ par rapport à l'axe central (2) et le corps d'enveloppe (25) présente une épaisseur radiale d, le rapport δ/d x 100% étant situé dans une gamme comprise entre 80% et 350%.

5. Implant intervertébral (1) selon l'une quelconque des revendications 1 à 4, **caractérisé en ce que** le système de fibres (5) est mobile par rapport au corps d'enveloppe (25).

6. Implant intervertébral (1) selon l'une quelconque des revendications 1 à 4, **caractérisé en ce que** le système de fibres (5) est positionné de manière immobile par rapport au corps d'enveloppe (25).

7. Implant intervertébral (1) selon l'une quelconque des revendications 1 à 6, **caractérisé en ce que** l'ensemble du système de fibres (5) est relié aux plaques de recouvrement (3 ; 4).

8. Implant intervertébral (1) selon l'une quelconque des revendications 1 à 7, **caractérisé en ce que** le corps d'enveloppe (25) se compose d'un matériau élastique biocompatible, de préférence d'un élastomère, en particulier à base de polyuréthanne, ou de caoutchouc de silicone, de polyéthylène, de polycarbonate-uréthanne ou de poly(téréphtalate d'éthylène).

9. Implant intervertébral (1) selon l'une quelconque des revendications 1 à 8, **caractérisé en ce que** la partie centrale (10) est remplie au moins en partie d'un milieu incompressible.

10. Implant intervertébral (1) selon la revendication 9, **caractérisé en ce que** le milieu incompressible est un liquide.

11. Implant intervertébral (1) selon la revendication 10, **caractérisé en ce que** la partie centrale (10) comprend un noyau liquide incompressible (13) et un corps moulé élastique (9) disposé autour de celui-ci.

12. Implant intervertébral (1) selon l'une quelconque des revendications 1 à 11, **caractérisé en ce que** la partie centrale (10) présente une cavité (11).

13. Implant intervertébral (1) selon l'une quelconque des revendications 1 à 12, **caractérisé en ce que** le système de fibres (5) est ancré par voie mécanique sur ou dans les plaques de recouvrement (3 ; 4).

14. Implant intervertébral (1) selon l'une quelconque des revendications 1 à 12, **caractérisé en ce que** le système de fibres (5) est collé avec les plaques de recouvrement (3 ; 4).

15. Implant intervertébral (1) selon l'une quelconque des revendications 1 à 12, **caractérisé en ce que** la partie centrale (10) comprenant le système de fibres (5) intégré est reliée aux plaques de recouvrement (3 ; 4) par emboîtement.

16. Implant intervertébral (1) selon l'une quelconque des revendications 1 à 15, **caractérisé en ce que** le système de fibres (5) est formé par un fil continu, de préférence sous la forme d'un tricot ou d'un maillage.

17. Implant intervertébral (1) selon l'une quelconque des revendications 1 à 16, **caractérisé en ce que** chaque plaque de recouvrement (3 ; 4) présente sur sa périphérie une face latérale (21 ; 22) et des rainures (18) réparties sur la circonférence, pénétrant radialement dans les faces latérales (21 ; 22), et le système fibreux (5) peut être ancré dans ces rainures (18).

18. Implant intervertébral (1) selon l'une quelconque des revendications 1 à 17, **caractérisé en ce que** des canaux (19) sont formés dans les surfaces externes (7 ; 8) des plaques de recouvrement (3 ; 4) pour recevoir le système fibreux (5).

19. Implant intervertébral (1) selon l'une quelconque des revendications 1 à 18, **caractérisé en ce que** le système de fibres (5) est formé par un tissu.

20. Implant intervertébral (1) selon l'une quelconque des revendications 1 à 19, **caractérisé en ce que** la partie centrale (10) est conçue essentiellement en forme de cylindre creux, de prisme creux ou d'un corps de révolution, d'un ellipsoïde, d'un segment sphérique ou d'un tonneau ayant un axe de rotation coaxial à l'axe central (2).

21. Implant intervertébral (1) selon la revendication 19 ou 20, **caractérisé en ce que** le tissu est formé par des premières et deuxièmes fibres (6a/6b) et les premières fibres (6a) forment un angle α avec l'axe central (2) et les deuxièmes fibres (6b) forment un angle β avec l'axe central (2).

22. Implant intervertébral (1) selon la revendication 21, **caractérisé en ce que** les premières et deuxièmes fibres (6a ; 6b) sont entrelacées.

23. Implant intervertébral (1) selon l'une quelconque des revendications 11 à 22, **caractérisé en ce que** le corps moulé élastique (9) présente une surface de section transversale F_{F} orthogonale à l'axe central (2), la partie centrale (10) présente une surface de section transversale F_{M} orthogonale à l'axe central (2) et le rapport F_{F}/F_{M} de ces deux surfaces de section transversale est compris entre 30% et 65%.

24. Implant intervertébral (1) selon l'une quelconque des revendications 21 à 23, **caractérisé en ce que** l'angle α est compris entre 15° et 60°.

25. Implant intervertébral (1) selon l'une quelconque des revendications 21 à 24, **caractérisé en ce que** l'angle β est compris entre 15° et 60°.

26. Implant intervertébral (1) selon l'une quelconque des revendications 11 à 25, **caractérisé en ce que** le corps moulé élastique (9) est entouré d'une membrane semi-perméable et qu'une solution saline de préférence physiologique est contenue à l'intérieur du corps moulé élastique (9).

27. Implant intervertébral (1) selon l'une quelconque des revendications 1 à 26, **caractérisé en ce que** le système de fibres (5) est disposé en une seule couche par rapport à l'axe central (2).

28. Implant intervertébral (1) selon l'une quelconque des revendications 1 à 26, **caractérisé en ce que** le système de fibres (5) est disposé en plusieurs couches, de préférence en 2 à 6 couches par rapport à l'axe central (2).

29. Implant intervertébral (1) selon l'une quelconque des revendications 11 à 28, **caractérisé en ce que** le système de fibres (5) est enroulé sur le corps moulé élastique (9).

30. Implant intervertébral (1) selon la revendication 29, **caractérisé en ce que** le système de fibres (5) est enroulé dans deux sens différents sur le corps moulé élastique (9), de préférence selon une disposition avec une symétrie de rotation.

31. Implant intervertébral (1) selon l'une quelconque des revendications 1 à 30, **caractérisé en ce que** le système de fibres (5) se compose d'UHMWPE (polyéthylène à masse moléculaire très élevée).

32. Implant intervertébral (1) selon l'une quelconque des revendications 1 à 31, **caractérisé en ce qu'**une plaque terminale (14 ; 15), qui présente une surface (16 ; 17) externe, disposée transversalement à l'axe central (2), ayant une texture macroscopique, de préférence sous la forme de dents, peut être fixée sur chacune des plaques de recouvrement (3, 4).

33. Implant intervertébral (1) selon l'une quelconque des revendications 1 à 32, **caractérisé en ce que** les fibres présentent un diamètre dans une gamme comprise entre 0,005 mm et 0,025 mm.
